# EUROPEAN PATENT SPECIFICATION

(11) **EP 2 774 953 B1**
(45) Date of publication and mention of the grant of the patent: **27.06.2018**
(21) Application number: 13158442.7
(22) Date of filing: 08.03.2013
(51) Int. Cl.: C08L 71/02, A61F 9/00, A61K 31/00, A61K 9/00, A61K 47/36, C08J 3/075

(54) **Gelatable compositions**
Gelierbare Zusammensetzungen
Compositions pouvant être gélifiées

(43) Date of publication of application: 10.09.2014
(73) Proprietor: FLUORON GMBH, 89077 Ulm (DE); Dr. Schmidt Intraocularlinsen GmbH, 53757 St. Augustin (DE); Helmholtz-Zentrum Geesthacht Zentrum für Material- und Küstenforschung GmbH, 21502 Geesthacht (DE)
(72) Inventor: Neffe, Axel Thomas, 21502 Geesthacht (DE); Kamlage, Stefan, 21502 Geesthacht (DE); Diliprao, Santan Harshal, 21502 Geesthacht (DE); Lendlein, Andreas, 21502 Geesthacht (DE); Kugler, Wilfried, 89077 Ulm (DE); Messner, Arthur, 53757 St.Augustin (DE)
(74) Representative: Schiweck, Weinzierl & Koch Patentanwälte Partnerschaft mbB

(56) References cited:
- WO-A1-00/59516
- WO-A1-2011/100469
- JOANNA GAJEWIAK ET AL: "Aminooxy Pluronics: Synthesis and Preparation of Glycosaminoglycan Adducts", BIOMACROMOLECULES, vol. 7, no. 6, 1 June 2006 (2006-06-01), pages 1781-1789, XP055071380, ISSN: 1525-7797, DOI: 10.1021/bm060111b
- CHO-K-Y ET AL: "Release of ciprofloxacin from poloxamer-graft-hyaluronic acid hydrogels in vitro", INTERNATIONAL JOURNAL OF PHARMACEUTICS, ELSEVIER BV, NL, vol. 260, 1 January 2003 (2003-01-01), pages 83-91, XP002415575, ISSN: 0378-5173, DOI: 10.1016/S0378-5173(03)00259-X
- MALI MAHESH N ET AL: "Ion Activated In Situ Gel System for Ophthalmic Delivery of Moxifloxacin Hydrochloride", ACTA FARMACEUTICA BONAERENSE, COLEGIO DE FARMACEUTICOS DE LA PROVINCIA DE BUENOS AIRES, AR, vol. 29, no. 6, 1 September 2010 (2010-09-01), pages 876-882, XP008155835, ISSN: 0326-2383
- WEI G ET AL: "Thermosetting gels with modulated gelation temperature for ophthalmic use: the rheological and gamma scintigraphic studies", JOURNAL OF CONTROLLED RELEASE, ELSEVIER, AMSTERDAM, NL, vol. 83, no. 1, 18 September 2002 (2002-09-18), pages 65-74, XP004380060, ISSN: 0168-3659, DOI: 10.1016/S0168-3659(02)00175-X

## Description

### FIELD OF THE INVENTION

The present invention relates to ophthalmologic compositions having gelation ability that contain polysaccharides and modified mono and/or bi terminated amino di- or tri- block copolymers. More particularly, the compositions of the present invention are composition for use in replacing ophthalmologic structures. The compositions are useful as a vitreous body substitute, particularly a vitreous body that acts as an artificial vitreous body having a tamponade effect. Hence, the present compositions are useful in ophthalmologic surgery and in the treatment of diseases that require replacing ophthalmologic structures.

### BACKGROUND ART

The vitreous body is a jelly-like substance comprised of water, collagen and sodium hyaluronate that fills the posterior cavity of the eye. The vitreous body is a substance within the eye that is important in terms of its optical and dynamic characteristics. It stabilizes the bulbus and holds the retina in place. Age-related vitreous liquefaction leads to collapse of the vitreous body and as a result in vitreous detachment. If this process is incomplete, tractive forces may induce rhegmatogenous retinal detachment and sometimes vitreous hemorrhages. To treat these diseases effectively the vitreous body has to be removed, which is also necessary in disorders like proliferative diabetic retinopathy and proliferative vitreoretinopathy.

The removal of the vitreous (vitrectomy) is one of the standard techniques of vitreo-retinal surgery. The importance of these operations has increased in recent years because the functional results were significantly better than with alternative methods.

It is generally recognized, however, that the vitreous can be removed not only when there is the need to manipulate the retina, but also if the vitreous loses its optical properties because of hemorrhagic opacification. In addition unwanted traction of the retina, may possibly end up in retinal detachment with unfavorable rehabilitation diagnosis

Thus, it is important that the vitreous body role is considered in its interplay with the other functional units of the eye and in particular with the retina and the retinal pigment epithelium (RPE).

Vitrectomy makes research in adequate substitutes mandatory.

Silicone oils, perfluorocarbons and gases like sulfur hexafluoride (SF₆) are currently used as vitreous substitutes (endotamponades). Perfluorocarbons are mainly used during surgery because of severe side effects during long-term use. Silicone oils and gases are used as long-term tamponades. Whereas gases are absorbed over time silicone oils have to be removed surgically because of increased tendency of side effects, e.g. emulsification, inflammation, posterior capsule opacification, and the risk of secondary glaucoma

Thermo-sensitive gels are hydrogels for which at a change in the temperature a sol-gel transitions (usually reversible) is observed. There are quite a number of biological and synthetic polymers that show such a behavior. A distinction can be made mainly between (i) systems in which the sol-gel transition correlates with their (usually lower) critical solution temperature (LCST) and (ii) amphiphilic block copolymers that have a critical gelation temperature (CGT). In a third group of temperature-sensitive polymers, heating is used for the formation or elimination of physical interactions between different polymer chains, such as in the gelatin.

Poly (N-isopropylacrylamide) (PNIPAAM) and poly (N, N-diethylacrylamide) are typical examples of polymers having a LCST (in this case about 32 °C), that gels above this temperature. However a phase separation is observed that results an undesired volume contraction. This renders these polymers unsuitable for ophthalmic organ replacement. The exact value of the LCST of PNIPAAM-based thermo-sensitive polymers can be varied by copolymerization with N-vinylpyrrolidone (NVP).

Amphiphilic block copolymers often show the formation of micellar structures with increasing temperature, which can also lead to gelation. There occurs no, or only a small change in volume. A typical example of such a system is the aqueous solution of poly (ethylene oxide-block-propylene oxide-block-ethylene oxide (PEPE). The transition temperature of such a system is generally determined by the chain length (overall and of each block), the ratio between hydrophilic and hydrophobic blocks, and the concentration of the polymer. Interestingly, many ABA tri-block copolymers and block copolymers with further architectures (e.g. graft block copolymers) show a thermo-sensitive behavior.

However, at present no substance exists which simulates the properties of the vitreous body and sufficiently functions as a successful long-term or permanent vitreous body substitute.

The article by Cho-K-Y, et al., entitled "Release of ciprofloxacin from poloxamer-graft-hyaluronic acid hydrogels in vitro", International Jouranl of Pharmaceutics, describes a composition wherein hyaluronic acid is dissolved in water together with mono or bis-aminooxy Pluronic to give a thermo-geletable composition.

The article by Joanna Gajewiak et al., entitled "Aminooxy Pluronics: Synthesis and Preparation of Glycosaminoglycan Adducts", Biomacromolecules, describes a thermos-geletable composition for application of tissue engineering in ophthalmic drug delivery systems as well as for enhancing the wound healing of an injured mucus layer of the eye. The composition comprises graft copolymers prepared by coupling of mono- and bi terminated amino poloxamers with hyaluronic acid.

Patent document WO 2011/100469 A1 discloses a composition used to form a vitreous substitute comprising a hydrogel polymer containing: (a) oxidated hyaluronic acid and (b) a dihydrazide, wherein the dihydrazide cross-links the hyaluronic acid.

### SUMMARY OF THE INVENTION

The present invention provides new compositions that are a suitable material for ophthalmologic structure replacement in particular for vitreous body substitute having physical properties suited to eye tissue. The compositions of the invention are sol compositions that have gelation ability at certain temperature. Hence, the present sol compositions become a gel at certain temperatures.

The term "gelatable" is also used herein and means herein "capable of forming a gel".

The present invention hence provides a composition that can be injected and gelled in the eye in particular, in the vitreous body cavity, at a certain temperature.

The present inventors have found that a composition comprising polysaccharide (such as hyaluronic acid (HA) or derivative thereof) and, modified di or tri block-copolymer is a sol composition at room temperature and become a gel at a body temperature. The present inventors have therefore developed a gelatable composition that can be used in ophthalmologic organ replacement.

The gel composition toughness, the fluidity at the body temperature and the physical properties derived from the gelation of the sol composition of the invention are suited for eye tissue and for ophthalmologic structure replacement, in particular they are suited as vitreous body substitutes.

The present inventors have further found that with the gel compositions of the invention such as the vitreous body substitute, a reversal of gelation can be achieved by decrease of temperature, absorption of water, or can be supported by enzymatic or hydrolytic degradation of the hyaluronic acid part of the gel composition of the invention. Therefore, the vitreous body substitute of the invention can be removed on purpose as well as by natural dissolvation without the need of surgical removal.

The present invention was developed on the above basis.

Hence the present invention is directed to a gelifiable sol composition for use in ophthtalmic surgery, wherein the composition comprises: 1) polysaccharide having a deprotonated acid moiety, wherein preferably the polysaccharide is hyaluronic acid (HA) or a derivative thereof selected from the group consisting of esters, carbamates, amides and hyaluronic acid functionalized with 1-ethyl-3-(3-dimethylaminopropyl)carbodiimide hydrochloride (EDC); 2) mono and/or bi terminated amino tri block-copolymer selected from mono amino terminated poly(ethylene glycol)-block-poly(propylene glycol)-block-poly(ethylene glycol), bi amino terminated poly(ethylene glycol)-block-poly(propylene glycol)-block-poly(ethylene glycol) or mixture thereof, wherein an amino group of the tri block-copolymer is protonated; and 3) water or physiological saline or an aqueous buffer system.

Component 2) i.e. mono and/or bi terminated amino tri block-copolymer in any embodiment of the invention is selected from mono amino terminated poly(ethylene glycol)-*block*-poly(propylene glycol)-*block*-poly(ethylene glycol), bi amino terminated poly(ethylene glycol)-*block*-poly(propylene glycol)-*block*-poly(ethylene glycol) or mixture thereof. They are also indicated as mono and/or bi terminated amino PEPEs. The mono and/or bi terminated amino PEPEs of the invention preferably have a Mₙ that ranges from about 10000 to about 17000 g mol⁻¹, preferably about 14000 to 15000 g mol⁻¹, more preferably 14500-14700, even more preferably 14600 g mol⁻¹.

Preferably, the mono terminated amino poly(ethylene glycol)-*block*-poly(propylene glycol)-*block*-poly(ethylene glycol) is of formula (I) 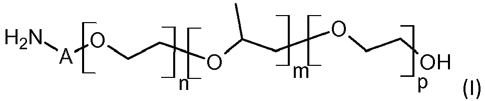 wherein
A is selected from -CO-C₂-C₆alkylene or -CO-C₂-C₆alkylene-NH-C₂-C₆alkylene-, preferably C₂-C₆ alkylene is propylene or ethylene wherein in A the -CO- group is linked to the polymer part of formula (I), preferably, NH₂A is H₂N propylene-NH-ethylene-CO-
n is independently an integer from 130-150, preferably 139-145, more preferably 140 or 141;
m is independently an integer from 40 to 50, preferably 43-46 more preferably 44;
p is independently an integer from 130-150, preferably 139-145, more preferably 140 or 141; the NH₂ group is protonated; and n, m and p are the average mole numbers;
or of formula (I*) 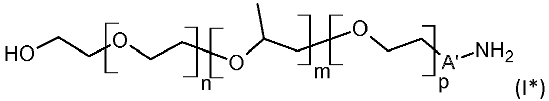 wherein
A' is selected from -O-CO-C₂-C₆ alkylene or -O-CO-NH-C₂-C₆alkylene-, preferably C₂-C₆ alkylene is propylene or ethylene, wherein in A' the -O-CO- group is linked to the polymer part of formula (I*), preferably NH₂A'- is H₂N-propylene-NH-OC-O-
n is independently an integer from 130-150, preferably 139-145, more preferably 140 or 141;
m is independently an integer from 40 to 50, preferably 43-46 more preferably 44;
p is independently an integer from 130-150, preferably 139-145, more preferably 140 or 141; the NH₂ group is protonated; and n, m, and p are the average mole numbers;
and the bi-terminated amino poly(ethylene glycol)-*block*-poly(propylene glycol)-*block*-poly(ethylene glycol) is of formula (II) 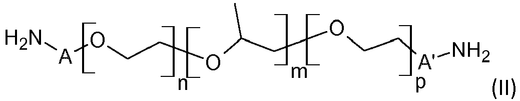 wherein
A is selected from -CO-C₂-C₆ alkylene or -CO-NH-C₂-C₆alkylene-, preferably C₂-C₆ alkylene is propylene or ethylene, wherein in A the -CO- group is linked to the polymer part of formula (II), NH₂A is H₂N-propylene-NH-ethylene-CO-
A' is selected from -O-CO-C₂-C₆ alkylene, -O-CO-NH-C₂-C₆alkylene-, preferably C₂-C₆ alkylene is propylene or ethylene, wherein in A' the -O-CO- group is linked to the polymer part of formula (II), preferably NH₂A'- is H₂N-propylene-NH-OC-O-
n is independently an integer from 130-150, preferably 139-145, more preferably 140 or 141;
m is independently an integer from 40 to 50, preferably 43-46 more preferably 44;
p is independently an integer from 130-150, preferably 139-145, more preferably 140 or 141; the NH₂ group is protonated; and n, m and p are the average mole numbers;
or a mixture of compounds of formulae (I), (I*) and (II).

Component 2) can also be a mixture of compounds of formulae (I) (I*) and (II).

In a further preferred embodiment the composition according to the invention is wherein component 2) is of formula (I') 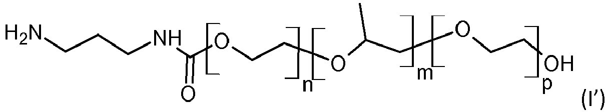 or of formula (I") 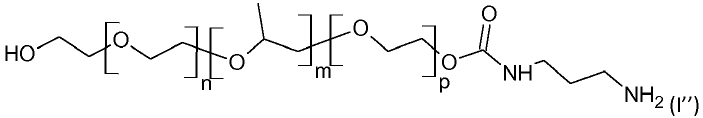 or of formula (II') 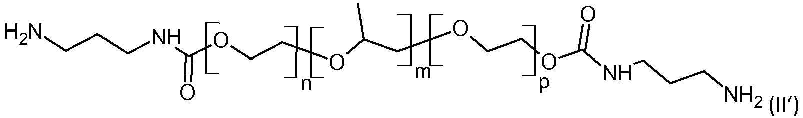 wherein in formulae (I') (I") and (II')
n is independently an integer from 130-150, preferably 139-145, more preferably 140 or 141;
m is independently an integer from 40 to 50, preferably 43-46 more preferably 44;
p is independently an integer from 130-150, preferably 139-145, more preferably 140 or 141; an NH₂ group is protonated; and wherein n, m and p indicate the respective average number of moles,
or a mixture of compounds of formulae (I') (I") and (II').

Preferred components 2) are compounds of formula (I') (I") and (II') wherein n and p are 140 or 141 and m is 44.

Component 2) can also be a mixture of compounds of formula (I') (I") and (II').

In any embodiment of the inventions, the amino group of the mono and/or bi terminated amino tri block-copolymer exists in the composition of the invention in its protonated form (e.g., - NH₃⁺ in case of a protonated primary amino group) or in mixture of protonated and deprotonated form (e.g. -NH₃⁺ and -NH₂ in case of a primary amino group). The acid moiety (such as COOH) present on the polysaccharides component exists in the composition of the invention in its protonated form (e.g. COO⁻) or in mixture of deprotonated and protonated form (e.g. COO⁻ and COOH).

In an aspect, the gelatable sol composition of the present invention, comprises components 1) and 2) wherein the weight ratio of the component 2) to component 1) can fall within a range of 80: 20 to 97:3 preferably 93:7 to 96:4, more preferably 94:6 to 95:5, even more preferably 94.7:5.3.

The combined content of components 1 and 2) can range from 18% to 30% wt of the gelatable composition, preferably from the 20% to 25% wt.

The pH of the gelatable sol composition of the invention can range from 7 to 7.6, preferably from 7.1 to 7.4. A pH-adjusting agent can be further incorporated to the gelatable sol composition to achieve the desired pH. The pH adjusting agent can also be such that confers buffering properties to the composition of the invention.

The gelatable sol composition according to the invention is a thermo-gelatable composition. Preferbly, the transition between the sol to gel phase can occur at a temperature ranging from 28-37 °C, preferably ranging from 35-36°C.

The gelatable sol compositions as defined in any of the above embodiments are for use in ophthalmic surgery, in vitrectomy and in any disease treatment wherein a vitrectomy is required, e.g., retina detachment, macular pucker, diabetic retinopathy, macular holes, vitreous hemorrhage and vitreous floaters.

The gelatable compositions of the invention can be used to prepare ophthalmic structure replacement, preferably vitreous body substitutes. Hence, the present invention is directed to ophthalmic structure replacement obtainable by gelation of the gelatable composition as defined in any of the above disclosed embodiments. Preferably, the gelation occurs at a temperature ranging from 28-37 °C, preferably ranging from 35-36°C. Preferably, the ophthalmic structure replacement is a vitreous body substitute.

Herein it is also disclosed a gel composition comprising or consisting of the composition as defined in any of the above embodiments. The gel composition is obtainable by gelation of the above disclosed gelatable compositions.

A fluorescent dye and/or coloring agent can be incorporated.

The gel composition of the present inventions can be employed as artificial vitreous bodies.

### DETAILED DESCRIPTION OF THE INVENTION

It has been seen by the present inventors that the modification of the ends of di or tri block-copolymer with amino group improves the physical properties of the gel composition that can be used as ophthalmologic structure replacement in particular as vitreous body substitute. In particular, the gel obtained using composition comprising a polysaccharide and the mono and/or bi terminated amino di or tri block-copolymers of the invention shows a stability, a transparency, an osmolality, a refractive index and biocompatibility that renders it suitable to be used as ophthalmologic structure replacement in particular as vitreous body substitute.

Without being bound to any theory, the present inventors believe that the amino group present on the block copolymers contribute to the stabilization of the gel. The terminal amino group in the composition is supposed to be protonated and the acid groups are deprotonated (e.g. -COO⁻), therefore an electrostatic interaction is postulated. The block-copolymers fold differently depending on the temperature, while at lower temperatures all parts are solubilized and a random coil is formed, with increasing temperature the middle block gets desolubilized, inducing a micelle formation (first step of the gelation). When enough micelles are formed, the micelles are packed ("colloidal jamming") leading to the macroscopic effect of sol-gel transition. As a consequence, the present inventors have found that the present composition behaves differently to the comparable blends of HA with hydroxy terminated PEPE. The explanation to that is the electrostatic interaction between the components of the composition of the invention, which supports the gelation and stability of the gel.

Herein it is also disclosed a composition comprising or consisting of:
1) polysaccharide(s), preferably, hyaluronic acid (HA) or derivative thereof, more preferably hyaluronic acid
2) mono and/or bi terminated amino di or tri block-copolymer(s).

"Hyaluronic acid" can be low or high molecular weight hyaluronic acid, for example the HA can range from 1 KDa to 5KDa, preferably from 1.5 to 4.5 KDa. HA derivatives can also be used. HA derivatives are for examples esters, carbamates, or amides. As a further example of HA derivatives, HA functionalized with 1-Ethyl-3-(3-dimethylaminopropyl)carbodiimide hydrochloride (EDC) can be mentioned.

Component 2) is a mono and/or bi terminated amino di or tri block-copolymer. The term "mono and be terminated" when referred to the di and tri block copolymer means that di and tri block copolymer has been modified such the one or both end of the polymer have been functionalized to bear an amino group. The amino group can be a primary secondary or tertiary amino group. Preferably it is a primary amino group. Di or tri block-copolymers are known in the art. For example "pluronic block polymers" are known tri block polymer such as Pluronic F-127, F-68. They are also known as polaxamer.

Component 2) is preferably selected from mono terminated amino poly(ethylene glycol)-*block*-poly(propylene glycol)-*block*-poly(ethylene glycol), bi terminated amino poly(ethylene glycol)-*block*-poly(propylene glycol)-*block*-poly(ethylene glycol) or mixture thereof. Poly(ethylene glycol)-*block*-poly(propylene glycol)-*block*-poly(ethylene glycol) is also indicated as PEPE. Mono amino PEPE is mono terminated amino poly(ethylene glycol)-*block*-poly(propylene glycol)-*block*-poly(ethylene glycol), bi amino PEPE is bi terminated amino poly(ethylene glycol)-*block*-poly(propylene glycol)-*block*-poly(ethylene glycol). The mono and/or bi terminated amino PEPEs of the invention preferably have a Mₙ that ranges from about 10000 to about 17000 g mol⁻¹, preferably about 14000 to 15000 g mol⁻¹, more preferably 14500-14700 g mol⁻¹, even more preferably 14600 g mol⁻¹.

Preferably, the mono terminated amino poly(ethylene glycol)-*block*-poly(propylene glycol)-*block*-poly(ethylene glycol) is of formula (I) 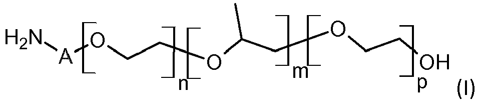 wherein
A is selected from -CO-C₂-C₆alkylene or -CO-C₂-C₆alkylene-NH-C₂-C₆alkylene-, preferably C₂-C₆alkylene is propylene or ethylene wherein in A the -CO- group is linked to the polymer part of formula (I), preferably NH₂A- is NH₂-propylene-NH-ethylene-CO-
n is independently an integer from 130-150, preferably 139-145, more preferably 140 or 141;
m is independently an integer from 40 to 50, preferably 43-46 more preferably 44;
p is independently an integer from 130-150, preferably 139-145, more preferably 140 or 141;
or of formula (I*) 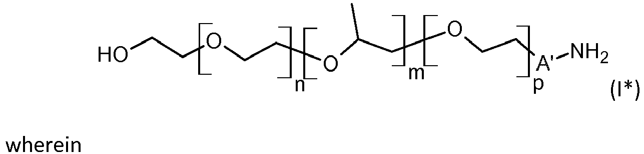
A' is independently selected from -O-CO-C₂-C₆alkylene or -O-CO-NH-C₂-C₆alkylene-, preferably C₂-C₆alkylene is propylene or ethylene, wherein in A' the -O-CO- group is linked to the polymer part of formula (I*), preferably NH₂A'- is NH₂-propylene-NH-CO-O-
n is independently an integer from 130-150, preferably 139-145, more preferably 140 or 141;
m is independently an integer from 40 to 50, preferably 43-46 more preferably 44;
p is independently an integer from 130-150, preferably 139-145, more preferably 140 or 141;
or of formula (II) 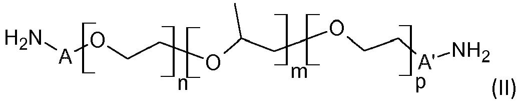 wherein
A is selected from -CO-C₂-C₆ alkylene or -CO-NH-C₂-C₆alkylene-, preferably C₂-C₆ alkylene is propylene or ethylene, wherein in A the -CO- group is linked to the polymer part of formula (II)
A' is selected from -O-CO-C₂-C₆ alkylene, -O-CO-NH-C₂-C₆alkylene-, preferably C₂-C₆ alkylene is propylene or ethylene, wherein in A' the -O-CO- group is linked to the polymer part of formula (II)
preferably H₂NA-is H₂N-propylene-NH-ethylene-CO-and/or H₂NA' is H₂N- propylene-NH-CO-O-
n is independently an integer from 130-150, preferably 139-145, more preferably 140 or 141;
m is independently an integer from 40 to 50, preferably 43-46 more preferably 44;
p is independently an integer from 130-150, preferably 139-145, more preferably 140 or 141;
or a mixture of compounds of formulae (I), (I*) and (II).

N, m and p in any formulae disclosed are the average mole numbers.

Preferably, in the compounds of formula (I) (I*) and (II) n is 140 or 141, m is 44 and p is 140 or 141.

Component 2) can also be a mixture of compounds of formula (I) (I*) and (II). Preferably, when component 2) is a mixture of compounds of formula (I) (I*) and (II) the component of formula (II) is present in a greater quantity with respect to the compound of formula (I) and (I*). Preferably, the compound of formula (II) is more than the 60% wt of the combined content of the compounds of formulae (I) (I*) and (II). Preferably, in the compounds of formula (I) (I*) and (II) of the mixture n is 140 or 141, m is 44 and p is 140 or 141. Herein it is also disclosed a composition wherein component 2) is of formula (I') 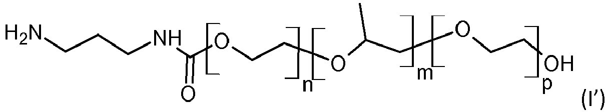 or of formula (I") 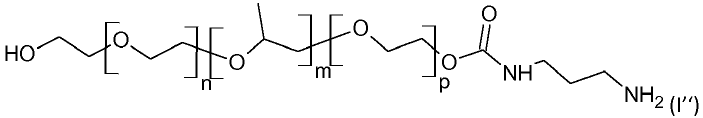 or of formula (II') 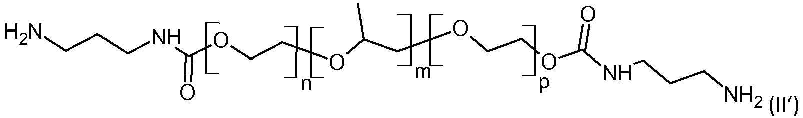 wherein in formulae (I') (I") and (II')
n is independently an integer from 130-150, preferably 139-145, more preferably 140 or 141;
m is independently an integer from 40 to 50, preferably 43-46 more preferably 44;
p is independently an integer from 130-150, preferably 139-145, more preferably 140 or 141; and wherein n, m and p indicate the respective average number of moles.
or a mixture of compounds of formulae (I') (I") and (II').

Preferred components 2) are compounds of formula (I') (I") and (II') wherein n is 140 or 141, m is 44 and p is 140 or 141.

Component 2) can also be a mixture of compounds of formula (I') (I") and (II'). Preferably, when component 2) is a mixture of compounds of formula (I') (I") and (II'), the component of formula (II') is present in a greater quantity with respect to the compound of formula (I') and (I"). Preferably the compound of formula (II) is more than the 60% wt of the combined content (100%) of the compounds of formula (I') (I") and (II'). Preferred components 2) are compounds of formula (I') (I") and (II') wherein n is 140 or 141, m is 44 and p is 140 or 141.

The present invention is directed to a gelifiable sol composition for use in ophthalmic surgery, wherein the composition comprises or consists of:
1) polysaccharide having a deprotonated acid moiety, wherein preferably the polysaccharide is hyaluronic acid (HA) or a derivative thereof selected from the group consisting of esters, carbamates, amides and hyaluronic acid functionalized with 1-ethyl-3-(3-dimethylaminopropyl)carbodiimide hydrochloride (EDC);
2) mono and/or bi terminated amino tri block-copolymer selected from mono amino terminated poly(ethylene glycol)-block-poly(propylene glycol)-block-poly(ethylene glycol), bi amino terminated poly(ethylene glycol)-block-poly(propylene glycol)-block-poly(ethylene glycol) or mixture thereof, wherein an amino group of the tri block-copolymer is protonated; and
3) water or physiological saline or an aqeuoues buffer system.

"Hyaluronic acid" according to the present invention can be low or high molecular weight hyaluronic acid, for example the HA of invention can range from 1 KDa to 5KDa, preferably from 1.5 to 4.5 KDa. HA derivatives can also be used according to the present invention. HA derivatives are selected from the group consisting of esters, carbamates, amides, or HA functionalized with 1-Ethyl-3-(3-dimethylaminopropyl)carbodiimide hydrochloride (EDC).

Component 2) is a mono and/or bi terminated amino tri block-copolymer. The term "mono and be terminated" when referred to the di and tri block copolymer means that di and tri block copolymer has been modified such the one or both end of the polymer have been functionalized to bear an amino group. The amino group can be a primary secondary or tertiary amino group. Preferably it is a primary amino group. Di or tri block copolymers are known in the art. For example "pluronic block polymers" are known tri block polymer such as Pluronic F-127, F-68. They are also known as poloxamer.

Component 2) is selected from mono terminated amino poly(ethylene glycol)-*block-*poly(propylene glycol)-*block*-poly(ethylene glycol), bi terminated amino poly(ethylene glycol)-b*lock-*poly(propylene glycol)-*block*-poly(ethylene glycol) or mixture thereof. Poly(ethylene glycol)-*block-*poly(propylene glycol)-*block*-poly(ethylene glycol) is also indicated as PEPE. Mono amino PEPE is mono terminated amino poly(ethylene glycol)-*block*-poly(propylene glycol)-*block*-poly(ethylene glycol), bi amino PEPE is bi terminated amino poly(ethylene glycol)-*block*-poly(propylene glycol)-*block*-poly(ethylene glycol). The mono and/or bi terminated amino PEPEs of the invention preferably have a Mₙ that ranges from about 10000 to about 17000 g mol⁻¹, preferably about 14000 to 15000 g mol⁻¹, more preferably 14500-14700 g mol⁻¹, even more preferably 14600 g mol⁻¹.

Preferably, the mono amino terminated poly(ethylene glycol)-*block*-poly(propylene glycol)-*block*-poly(ethylene glycol) is of formula (I) 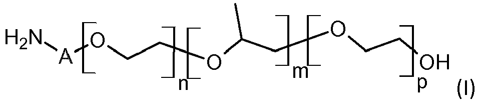 wherein
A is selected from -CO-C₂-C₆ alkylene or -CO-C₂-C₆ alkylene-NH-C₂-C₆alkylene-, preferably C₂-C₆ alkylene is propylene or ethylene wherein in A the -CO- group is linked to the polymer part of formula (I), preferably H₂NA-is H₂N-propylene-NH-ethylene-CO-
n is independently an integer from 130-150, preferably 139-145, more preferably 141;
m is independently an integer from 40 to 50, preferably 43-46 more preferably 44;
p is independently an integer from 130-150, preferably 139-145, more preferably 141; and the NH₂ group is protonated;
or of formula (I*) 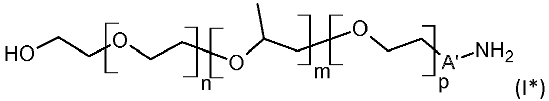 wherein
A' is independently selected from -O-CO-C₂-C₆ alkylene or -O-CO-NH-C₂-C₆alkylene-, preferably C₂-C₆ alkylene is propylene or ethylene, wherein in A' the -O-CO- group is linked to the polymer part of formula (I*), preferably NH₂A'- is NH₂-propylene-NH-CO-O-
n is independently an integer from 130-150, preferably 139-145, more preferably 140 or 141;
m is independently an integer from 40 to 50, preferably 43-46 more preferably 44;
p is independently an integer from 130-150, preferably 139-145, more preferably 140 or 141; and the NH₂ group is protonated;
or of formula (II) 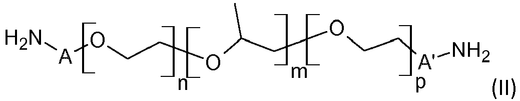 wherein
A is selected from -CO-C₂-C₆ alkylene or -CO-NH-C₂-C₆alkylene-, preferably C₂-C₆ alkylene is propylene or ethylene, wherein in A the -CO- group is linked to the polymer part of formula (II)
A' is selected from -O-CO-C₂-C₆ alkylene, -O-CO-NH-C₂-C₆alkylene-, preferably C₂-C₆ alkylene is propylene or ethylene, wherein in A' the -O-CO- group is linked to the polymer part of formula (II)
preferably H₂NA-is H₂N-propylene-NH-ethylene-CO-and/or H₂NA' is H₂N- propylene-NH-CO-O-
n is independently an integer from 130-150, preferably 139-145, more preferably 141;
m is independently an integer from 40 to 50, preferably 43-46 more preferably 44;
p is independently an integer from 130-150, preferably 139-145, more preferably 141; and the NH₂ group is protonated;
or a mixture of compounds of formulae (I), (I*) and (II).

N, m and p are the average mole numbers.

Preferably, in the compounds of formula (I) (I*) and (II) n is 140 or 141, m is 44 and p is 140 or 141.

Component 2) can also be a mixture of compounds of formula (I) (I*) and (II). Preferably, when component 2) is a mixture of compounds of formula (I) (I*) and (II), the component of formula (II) is present in a greater quantity with respect to the compound of formula (I). Preferably the compound of formula (II) is more than the 60% wt of the combined content of the compounds of formula (I) and (II). Preferably in the compounds of formula (I) (I*) and (II) of the mixture n is 141, m is 44 and p is 141.

In a further preferred embodiment, component 2) is of formula (I') 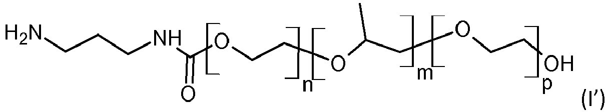 or of formula (I") 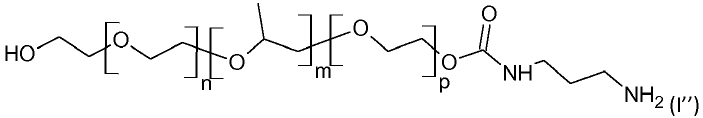 or of formula (II') 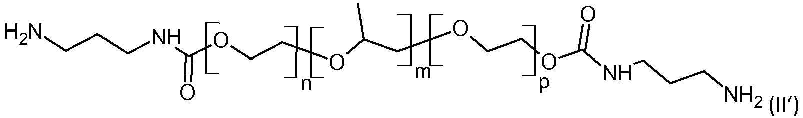 wherein in formulae (I') (I") and (II')
n is independently an integer from 130-150, preferably 139-145, more preferably 141;
m is independently an integer from 40 to 50, preferably 43-46 more preferably 44;
p is independently an integer from 130-150, preferably 139-145, more preferably 141; an NH₂ group is protonated; and wherein n, m and p indicate the respective average number of moles.
or a mixture of compounds of formulae (I') (I") and (II').

Preferred components 2) are compounds of formula (I') (I") and (II') wherein n is 140 or 141, m is 44 and p is 140 or 141.

Component 2) can also be a mixture of compounds of formula (I') (I") and (II'). Preferably, when component 2) is a mixture of compounds of formula (I') (I") and (II'), the component of formula (II') is present in a greater quantity with respect to the compound of formula (I') and (I"). Preferably, the compound of formula (II) is more than the 60% wt of the combined content (100%) of the compounds of formula (I') and (II'). Preferred components 2) are compounds of formula (I') and (II') wherein n is 140 or 141, m is 44 and p is 140 or 141.

In any embodiment of the inventions, the mono and/or bi terminated amino di or tri block-copolymer exists in its cationic form. The amino group of the mono and/or bi terminated amino tri block-copolymer of the composition of the invention exists in its protonated form (e.g., -NH₃⁺ in case of a protonated primary amino group) or in mixture of protonated form and non-protonated form (e.g., -NH₂ and -NH₃⁺ in case of a primary amino group). In any embodiment of the inventions, the polysaccharide exists in its anionic form. An acid moiety (such as COOH) present on the polysaccharides component exists in the composition of the invention as deprotonated (such as COO⁻ ) or in mixture of deprotonated and protonated (such as COO⁻ and COOH). Hence for example, the compositions of the invention are also compositions wherein the amino group "NH₂" of formulae (I) (I*) (II) (I') (I") and (II') is protonated. Hence, the compositions of the invention are also compositions wherein the polysaccharide is in an anionic form. The skilled person understands that ionic form and non ionic form can coexist and can coexist in a different amount in dependence of the pH of the composition of the invention.

In an aspect the gelatable sol composition of the present invention, comprises components 1) and 2) wherein the weight ratio of the component 2) to component 1) can fall within a range of 80: 20 to 97:3 preferably 93:7 to 96:4, more preferably 94:6 to 95:5, even more preferably 94.7:5.3.

The combined content of components 1 and 2) can range from 18% to 30% wt of the gelatable composition, preferably from the 20% to 25% wt of the gelatable composition; even more preferably the 20% wt of the gelatable composition.

Hence, for example the composition of the invention may comprise HA and mono and/or bi terminated amino PEPE wherein the weight ratio HA: mono and/or bi terminated amino PEPE is 94.7:5.3 and their content in the gelatable composition of the invention is of 20% wt of the gelatable composition.

The pH of the gelatable composition of the invention can range from 7 to 7.6 preferably from 7.1 to 7.4. A pH-adjusting agent can be further incorporated to the gelatable sol composition to achieve the desired pH. pH adjusting agents suitable for the purpose are known in the art. They can be for examples, a base such as NaOH, or a buffer system such as mono sodium and/or disodium phosphate, bicarbonates, urea, salts of weak basis, alkali metal salt of boric acid etc.

The pH adjusters are such that confer the desired pH to the gelatable composition of the invention, they can also impart a pH buffering effect to the composition of the present invention.

Tonicity adjuster(s) can also be included to give an optimal tonicity and confers optimal osmolality to the gel sol composition of the invention. Tonicity adjusters for ophthalmic solutions are known in the art.

Additives such as microbiological preservative and ophthalmologic excipients such as EDTA can also be present in the gelatable sol composition.

Un-functionalized di or tri block-copolymer such as poly(ethylene glycol)-*block-*poly(propylene glycol)-*block*-poly(ethylene glycol) can also be present in the gelatable sol composition of the invention.

The gelatable sol composition according to the invention is a thermo-gelatable composition. Preferably, the transition between the sol to gel phase occurs at a temperature ranging from 28-37 °C, preferably ranging from 35-36°C.

In a preferred embodiment, the gelatable sol composition of the invention is a composition comprising
1) HA;
2) compounds of formulae (I') and/or (I") and/or (II');
3) water
4) a buffer system.

Preferably, in this preferred embodiment, the pH of the sol composition is of 7.2-7.4. Components 1) and 2) may be present totally or partially in their ionic forms. Preferably, component 2) is the 18%-21% of the sol composition, more preferably the 20% wt of the sol composition. Preferably, the weight ratio of component 2) and component 1) is ranging from 80: 10 to 97:3 preferably 93:7 to 96:6, more preferably 94:6 to 95:5, even more preferably 94.7:5.3.

In a further preferred embodiment the gelatable sol composition of the invention is a composition comprising
1) HA
2) compounds of formulae (I) and/or (I*) and (II)
3) water
4) a buffer system.

Preferably, in this preferred embodiment, the pH of the sol composition is of 7.2-7.4. Components 1) and 2) may be present totally or partially in their ionic forms. Preferably, component 2) is the 18%-21% wt of the sol composition, more preferably the 20% wt of the sol composition. Preferably, the weight ratio of component 2) and component 1) is ranging from 80: 10 to 97:3 preferably 93:7 to 96:6, more preferably 94:6 to 95:5, even more preferably 94.7:5.3.

In a further preferred embodiment the gelatable sol composition of the invention is a sol composition comprising
1) HA
2) mono and/or bi terminated amino PEPE having a Mₙ that ranges from about 10000 to about 17000 g mol⁻¹, preferably about 14000 to 15000 g mol⁻¹, more preferably 14500-14700, even more preferably 14600 g mol⁻¹
3) water
4) a buffer system.

Preferably, the pH of the sol composition is of 7.2-7.4. Component 1) and 2) may be present totally or partially in their ionic forms. Preferably, component 2) is the 18%-21% wt of the sol composition, more preferably the 20% wt of the sol composition. Preferably, the weight ratio of component 2) and component 1) is ranging from 80: 10 to 97:3 preferably 93:7 to 96:6, more preferably 94:6 to 95:5, even more preferably 94.7:5.3.

The gelatable sol composition as defined in any of the above embodiment are for use in ophthalmic surgery preferably in vitrectomy and in any disease treatment wherein a vitrectomy is required, e.g., retina detachment, macular pucker, diabetic retinopathy, macular holes, vitreous hemorrhage and vitreous floaters.

The gelatable composition of the invention can be used to prepare ophthalmic structure replacement, preferably vitreous body substitutes. Hence the present invention is directed to ophthalmic structure replacement obtainable by gelation of the gelatable composition as defined in any of the above disclosed embodiments. Preferably, the gelation occurs at a temperature ranging from 28-37 °C, preferably ranging from 35-36°C. Preferably, the ophthalmic structure replacement is a vitreous body substitute.

The vitreous body substitute can be prepared by injection of the gelatable solution of the invention which is as as disclosed in any of the above embodiments of the invention, in the vitreous body cavity. The gelatable solution is then gelled by the heating of the body temperature. For example, the gelatable solution can be injected by means of a syringe or any suitable means. Syringe having a 27G needle can be used for injecting the gelatable solution of the invention into the cavity.

Herein it is also disclosed a gel composition comprising or consisting of the compositions as defined in any of the above embodiments. The gel composition is obtainable by gelation of the above disclosed gelatable compositions.

A dye such as fluorescent dye, and/or coloring agent can be incorporated in the gelatable composition of the invention. The incorporation of dyes and coloring agents can be used to impart visibility to the interior of the eye. The fluorescent dyes and coloring agents that are employed are preferably trypan blue, diazo and azo-dye, triphenylmethane-dye such as brilliant blu G, brilliant blue R and Patent blue V, cyanine-based dye and naphthocyanine based dye such as those disclosed in WO2011151287 and natural dyes. Hence the gel and the gelifiable composition of the invention may comprise dye such as fluorescent dye, and/or coloring agent such as trypan blue, diazo and azo-dye, triphenylmethane-dye such as brilliant blu G, brilliant blue R and Patent blue V, cyanine-based dye and naphthocyanine based dye such as those disclosed in WO2011151287 and natural dyes.

Drugs such as antibiotics (cyclosporine A) or VEGF inhibitors and other, vitamins, minerals, and antioxidant can be incorporated in the gelatable sol composition of the invention.

The gelatable sol composition of the present invention can be employed for the preparation of an artificial vitreous body. Once gelled, the sol compositions of the invention are artificial vitreous body substitute.

Herein it is also disclosed a kit comprising the gelatable composition of the invention. The present kit can comprise a syringe and a container containing the gelatable composition of the invention. Alternatively the syringe may contain the gelatable composition of the invention.

Alternatively, the kit may comprise the gelifiable sol composition comprising components 1) and 2). The composition may be then diluted with water or saline or a suitable buffer system to achieve the gelatable composition of the invention. The kit may further comprise a syringe.

The present invention is further directed to a method for preparing an ophthalmic replacement structure such as artificial vitreous body which comprises injecting the gelatable sol composition of the invention in a vitreous cavity after removal of natural vitreous body. The gel is formed due to the body temperature

The method may further comprises the removal of the ophthalmic replacement structure such as artificial vitreous body by decrease of temperature or by absorption of water, or by enzymatic or hydrolytic degradation of the hyaluronic acid part of the gel composition of the invention.

Artificial vitreous body and vitreous body substitute are herein used inter-changeably

Polysaccharides as used herein are long carbohydrate molecules of repeated monomer units joined together by glycosidic bonds. They range in structure from linear to highly branched. Polysaccharides are often quite heterogeneous, containing slight modifications of the repeating unit. Depending on the structure, these macromolecules can have distinct properties from their monosaccharide building block. Any polysaccharide, with suitable viscosity, molecular mass and other desirable properties may be utilized in the composition of the present invention.

By glycosaminoglycan is intended any glycan (i. e., polysaccharide) comprising an unbranched polysaccharide chain with a repeating disaccharide unit, one of which is always an amino sugar. These compounds as a class carry a high negative charge, are strongly hydrophilic, and are commonly called mucopolysaccharides. This group of polysaccharides includes heparin, heparan sulfate, chondroitin sulfate, dermatan sulfate, keratan sulfate, and hyaluronic acid. Hyaluronic acid is one of the preferred components of the invention.

It must be noted that as used herein, the singular forms "a", "an", and "the", include plural references unless the context clearly indicates otherwise. Thus, for example, reference to "a reagent" includes one or more of such different reagents and reference to "the method" includes reference to equivalent steps and methods known to those of ordinary skill in the art that could be modified or substituted for the methods described herein.

All publications and patents cited in this disclosure are incorporated by reference in their entirety. To the extent the material incorporated by reference contradicts or is inconsistent with this specification, the specification will supersede any such material.

Unless otherwise indicated, the term "at least" preceding a series of elements is to be understood to refer to every element in the series. Those skilled in the art will recognize, or be able to ascertain using no more than routine experimentation, many equivalents to the specific embodiments of the invention described herein. Such equivalents are intended to be encompassed by the present invention.

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or sometimes when used herein with the term "having".

When used herein "consisting of" excludes any element, step, or ingredient not specified in the claim element. When used herein, "consisting essentially of" does not exclude materials or steps that do not materially affect the basic and novel characteristics of the claim.

In each instance herein any of the terms "comprising", "consisting essentially of" and "consisting of" may be replaced with either of the other two terms.

Several documents are cited throughout the text of this specification. Each of the documents cited herein (including all patents, patent applications, scientific publications, manufacturer's specifications, instructions, etc.), whether supra or infra, are hereby incorporated by reference in their entirety. Nothing herein is to be construed as an admission that the invention is not entitled to antedate such disclosure by virtue of prior invention.

As described herein, "preferred embodiment" means "preferred embodiment of the present invention". Likewise, as described herein, "various embodiments" and "another embodiment" means "various embodiments of the present invention" and "another embodiment of the present invention", respectively.

### FIGURES

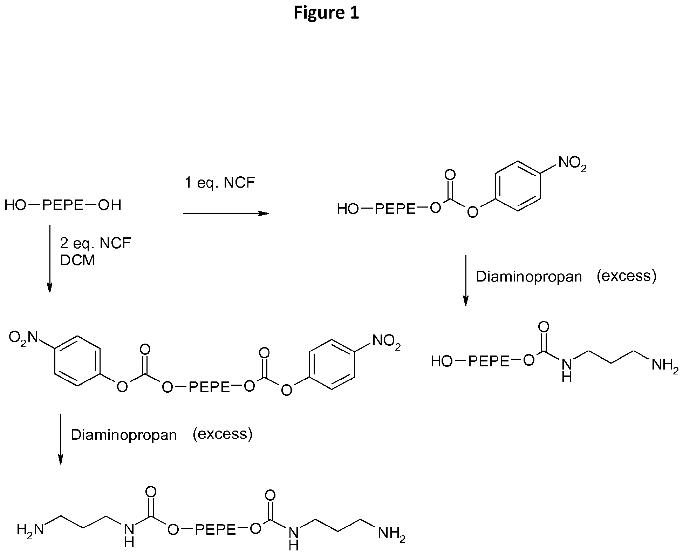

Figure 1: Scheme of the synthesis of mono and bi amino terminated PEPE.
Figure 2: Rheological investigation of the mixture of HA with mono amino PEPE. The values for the viscosity are shown in orange (η*), red is the viscous modulus (G') and the elastic modulus shown in blue (G").
Figure 3: Representation of the phases of micellization and gelation by a logarithmic plot of G 'and G" over the temperature range of 20-35 °C on the sample of HA (1.6KDa)/mono amino PEPE (3.4:96.4)
Figure 4: Rheological analysis of HA/diamino-PEPE.

### EXAMPLES

### Materials.

The hyaluronic acid of 2.6 MDa HA, Poly(ethylene glycol)-*block*-poly(propylene glycol)-*block-*poly(ethylene glycol) (PEPE, Mₙ ∼14,600 g·mol⁻¹) from Sigma Aldrich (Schnelldorf, Germany), 4-nitrophenyl chloroformate (4NCP), 1,3-diaminopropane (DAP), triethylamine, hexane, dichloromethane (DCM), and 1-Ethyl-3-(3-dimethylaminopropyl)carbodiimid hydrochloride (EDC) were used without further purification.

### Example 1: Synthesis of mono-amine terminated PEPE (1) Compound 1.

12 g (0.816 mmol) of PEPE was dissolved in 200 ml dichloromethane, 4-nitrophenyl chloroformate (198 mg, 0.979 mmol) was added in the presence of triethylamine (0.113 ml, 0.979 mmol) at room temperature. The reaction was stirred overnight and precipitated in 8 fold hexane. The vacuum dried intermediate was dissolved in 200 ml dichloromethane and reacted with excess of diaminopropane (0.206 ml, 2.44 mmol) overnight stirring at room temperature, and precipitated in 8 fold hexane. The precipitated product was dried under vacuum at 40 °C for 24 hrs. It was re-dissolved in dichloromethane and passed through an alumina (Al₂O₃) column to remove the side-product (p-nitrophenol), re-precipitated in 8 fold hexane and vacuum dried before use.

### Example 2: Synthesis of bi-amine terminated PEPE Compound 2

After dissolving 12 g (0.816 mmol) PEPE in 200 ml dichloromethane, an excess of 4-nitrophenyl chloroformate (493 mg, 2.448 mmol) was added in the presence of triethylamine (0.283 ml, 2.448 mmol) at room temperature. The reaction mixture was stirred overnight and precipitated in 8 fold hexane. The vacuum dried intermediate was dissolved in 200 ml dichloromethane and reacted with excess of diaminopropane (0.274 ml, 3.26 mmol) overnight at room temperature. Further workup was identical with workup of compound 1 of example 1.

### Example 3: Mixing of HA and mono-amine terminated PEPE (1) or bi-amine terminated PEPE (2).

In a typical procedure, 108 mg hyaluronic acid was dissolved in 100 mL water under stirring overnight. Then, the pH was adjusted to 4.75 by adding 0.1 M HCl (aq). Subsequently, 25 mg *N*-(3-Dimethylaminopropyl)-N'-ethyl carbodiimid Hydrochloride was added and the pH-value readjusted to 4.75. 4.0 g of **1** or 263 mg of **2** was added and the reaction mixture was stirred for 2.5 h while monitoring/readjusting the pH to 4.75. After stirring overnight, the reaction mixture was transferred into dialysis tubes with a molecular weight cut-off of 25000 Da and dialyzed against water for 96 h under constant change of the water. Finally, the material was freeze dried giving 3.7 g of the mixture of **1** and HA (93%).

### Example 4: Preparation of the gel.

400 mg of the material (compound 2 and HA) was dissolved in 1.6 g steam sterilized buffer buffer (1.9 mg Na₂HPO₄ ·2H₂O and 0.3 mg NaH₂PO4 2H₂O per 1 mL of water, adjusted to pH 7.4 by adding 0.1 M aq. NaOH) by rotating the vial overnight.

### Example 5: Rheological measurement of HA/monoamino PEPE composition.

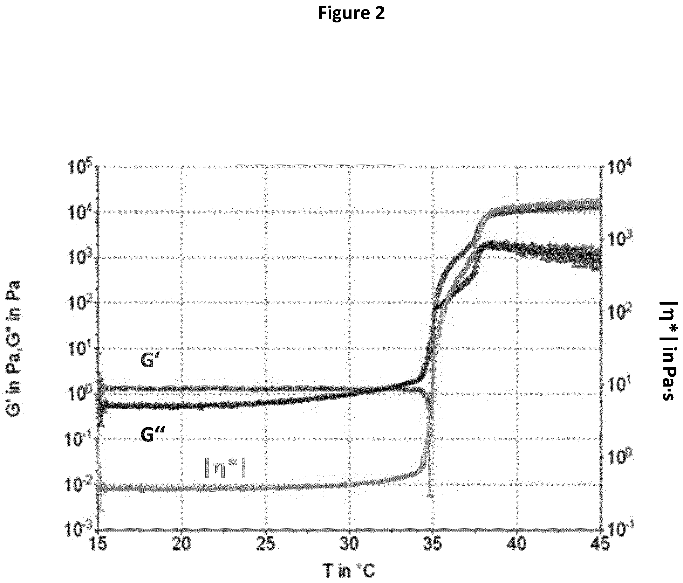

The rheological properties of the composition of HA/monoamino PEPE gelatable composition have been measured. (Fig. 2)

The sol-gel transition of the composition of example 4 was studied in detail by rheological measurements at the temperature change. An example curve is shown in Figure 2. The transition temperature, the values of the viscous modulus G', the elastic modulus G", and the viscosity |η*| are significantly lower than above this temperature (35 °C).

### Example 6:

**Table 1**

| Mₙ (HA) | HA: PEPE | | T_{gel} | T_{gel} Interval | Hys. | G'^{a} | G"^{a} | \|η*\|^{a} | G'^{b} | G"^{b} | \|η*\|^{b} | Konz. |
|---|---|---|---|---|---|---|---|---|---|---|---|---|
| [MDa] | [wt.-%] | | [°C] | [°C] | [°C] | [Pa] | [Pa] | [Pa·s] | [Pa] | [Pa] | [Pa·s] | [wt.-%] |
| 2.33 | 5.1 | 94.9 | 29 | 4 | 10 | 1.268 | 1.265 | 0.439 | 12440 | 1370 | 3063 | 20 |
| 2.33 | 8.2 | 91.8 | 29 | 4 | 12 | 1.61 | 1.844 | 0.599 | 9297 | 565 | 2281 | 20 |
| 1.6 | 4.6 | 95.4 | 31 | 3 | n.d. | 1,39 | 0.915 | 0.441 | 12330 | 584 | 3275 | 20 |
| 1.6 | 3.4 | 96.4 | 31 | 3 | n.d. | 1.054 | 0.851 | 0,359 | 13370 | 642 | 3552 | 20 |

### Example 7: Monitoring of the micellization and gelation phase of a composition of the invention.

The micellization and the gelation of composition according to the invention have been monitored.

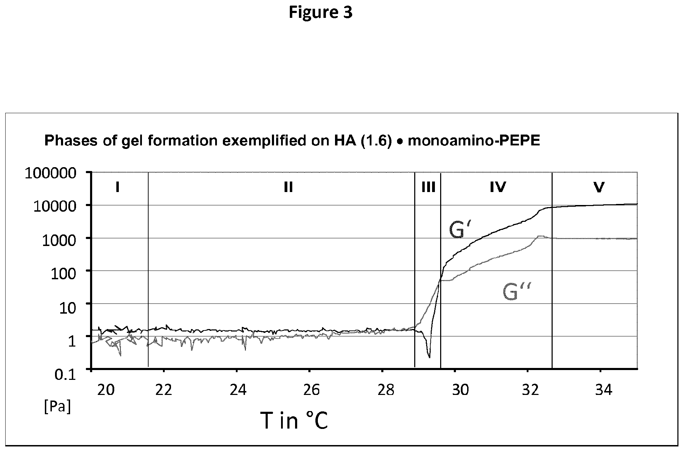

The composition of the invention contains HA having a molecular weight of 1.6 KDa and a mono-amino PEPE. The ratio HA:monoamino-PEPE is 4.4:96.4. The results are reported in Figure 3.

The plot of figure 3 is divided in five sections according to the temperature. In all five sections the micellization and gelation of HA/PEPE monoamine composition were monitored. In Section I, all monomers are in solution and have formed no higher structures. In Section II, a flat increase of the loss module is obderved. Then in Section III, a sharp increase in the loss modulus (G") until it reaches a plateau is observed. In this temperature range, the memory module (G') has a local minimum. It is known that in solution a PEPE forms a bilayer at the interface. Inside introverted PEG there are strong hydrophilic interactions, so that a large surface tension arises. This surface tension is likely to be increased by the electrostatic interactions and could therefore from the beginning of Section III dominate significantly the memory module. Within the short temperature interval of Section III to a strong micellization occurs. From the temperature T_{gel} the formed structures strongly interact and this allows the memory and loss moduli to increase rapidly. At the end of Section III all PEPE are bound in structures and a higher macromolecular structure arrangement begins to form in Section IV. A clear indication of a two-stage gelation is the flattening of the memory modulus and the plateau-like simultaneous stagnation of the loss modulus in the temperature curve. In Section V, memory and loss moduli stabilize so that it is recognizable that the gelation is complete.

### Example 8: HA and diamino PEPE.

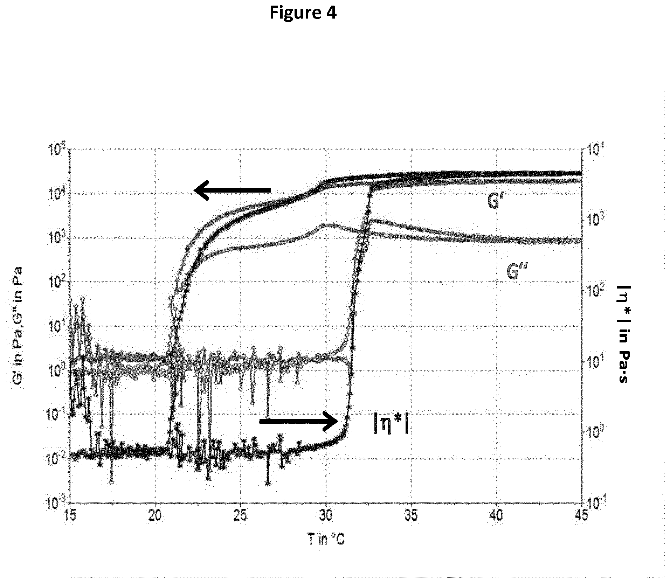

Rheological analysis of the sol-gel transition was performed. Figure 4 shows an example of the thermo-mechanical behavior during heating and cooling of the gel. A pronounced hysteresis was observed. The sol-gel transition is sharper than the gel-sol transition, and is also sharper than the HA/PEPE monoamine systems.

### Table 4 shows the thermo-mechanic properties of HA/diamino terminated PEPE.

**Table 4**

| Mₙ (HA) | HA: PEPE | | T_{gel} | T_{gel} Interval | Hys. | G'^{a} | G"^{a} | \|η*\|^{a} | G'^{b} | G"^{b} | \|η*\|^{b} | Konz. |
|---|---|---|---|---|---|---|---|---|---|---|---|---|
| [MDa] | [wt.-%] | | [°C] | [°C] | [°C] | [Pa] | [Pa] | [Pa·s] | [Pa] | [Pa] | [Pa·s] | [wt.-%] |
| 2.33 | 4.5 | 95.5 | 34 | 2 | 7 | 2.395 | 0.507 | 0.599 | 15580 | 1176 | 3825 | 16 |
| 1.2 | 5.5 | 94.5 | 29 | 3 | 8 | 1.202 | 0.997 | 0.382 | 21280 | 940 | 5216 | 20 |

| | | | | | | | | | | | | |
|---|---|---|---|---|---|---|---|---|---|---|---|---|
| a: below T_{gel}; b: above T_{gel}. | | | | | | | | | | | | |

The investigation of composition of HA with diamino terminated PEPE showed similar results with respect to the HA/monoamino terminated PEPE. Thermo-sensitive gels were obtained, the T_{gel} and the mechanical properties above the T_{gel} could be set by varying the concentration and the ratio of the components. The transition was slightly sharper and was also observed at lower concentrations. Furthermore, on the systems studied here, a higher stability against dilution experiments is observed. The composition of HA (2.33kDa)-diamino-PEPE (4.5:95.5) showed all the desired physical parameters that were set including the biocompatibility, the long-term stability with respect to temperature, and ultraviolet light, transparency and refractive index.

### INDUSTRIAL APPLICABILITY

The ophthalmologic composition of the present invention is not toxic to cells, is transparent, has a stable structure, is injected and removed rapidly by changing the temperature, has physical properties suited to eye tissue with a tamponade effect, and is useful as a material for replacing ophthalmologic structures.

## Claims

1. Gelifiable sol composition for use in ophthalmic surgery, wherein the composition comprises :
1) Polysaccharide having a deprotonated acid moiety, wherein preferably the polysaccharide is hyaluronic acid (HA) or a derivative thereof selected from the group consisting of esters, carbamates, amides and hyaluronic acid functionalized with 1-ethyl-3-(3-dimethylaminopropyl)carbodiimide hydrochloride (EDC);
2) mono and/or bi terminated amino tri block-copolymer selected from mono amino terminated poly(ethylene glycol)-*block*-poly(propylene glycol)-*block*-poly(ethylene glycol), bi amino terminated poly(ethylene glycol)-*block*-poly(propylene glycol)-*block-*poly(ethylene glycol) or mixture thereof, wherein an amino group of the tri block-copolymer is protonated; and
3) water or physiological saline or an aqueous buffer system.

2. The composition according to claim 1 wherein components 1) and 2) together are 18% to 30% wt, preferably 18% to 25% wt, more preferably 20% wt of the composition.

3. The composition according to any of the preceding claims wherein components 2) and 1) are in a weight ratio ranging from 80:20 to 97:3, preferably 93:7 to 96:4, more preferably 94:6 to 95:5, even more preferably 94.7:5.3.

4. The composition according to any of the preceding claims wherein component 2) is of formula 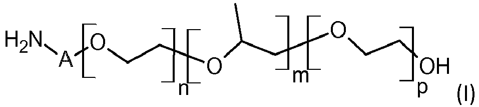 wherein
A is selected from -CO-C₂-C₆ alkylene or -CO-C₂-C₆ alkylene-NH-C₂-C₆alkylene-, preferably C₂-C₆ alkylene is propylene or ethylene and wherein in A the -CO- group is linked to the polymer part of formula (I);
n is independently an integer from 130-150, preferably 139-145, more preferably 141;
m is independently an integer from 40 to 50, preferably 43-46 more preferably 44;
p is independently an integer from 130-150, preferably 139-145, more preferably 141; the NH₂ group is protonated and n, m and p are the average mole numbers;
or of formula (I*) 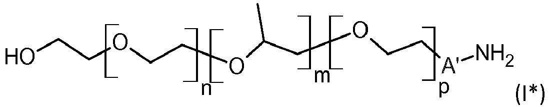 wherein
A' is independently selected from -O-CO-C₂-C₆ alkylene or -O-CO-NH-C₂-C₆alkylene-, preferably C₂-C₆ alkylene is propylene or ethylene, wherein in A' the -O-CO- group is linked to the polymer part of formula (I*);
n is independently an integer from 130-150, preferably 139-145, more preferably 141;
m is independently an integer from 40 to 50, preferably 43-46 more preferably 44;
p is independently an integer from 130-150, preferably 139-145, more preferably 141; the NH₂ group is protonated and n, m and p are the average mole numbers;
or of formula (II) 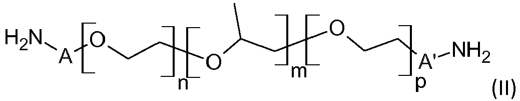 wherein
A is selected from -CO-C₂-C₆ alkylene or -CO-NH-C₂-C₆alkylene-, preferably C₂-C₆ alkylene is propylene or ethylene, wherein in A the -CO- group is linked to the polymer part of formula (II);
A' is selected from -O-CO-C₂-C₆ alkylene, -O-CO-NH-C₂-C₆alkylene-, preferably C₂-C₆ alkylene is propylene or ethylene, wherein in A' the -O-CO- group is linked to the polymer part of formula (II);
n is independently an integer from 130-150, preferably 139-145, more preferably 141;
m is independently an integer from 40 to 50, preferably 43-46 more preferably 44;
p is independently an integer from 130-150, preferably 139-145, more preferably 141; the NH₂ group is protonated and n, m and p are the average mole numbers;
or a mixture of compounds of formulae (I), (I*) and (II).

5. The composition according to any of the preceding claims wherein component 2) is of formula (I') 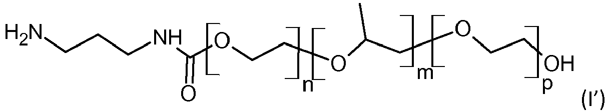 or of formula (I") 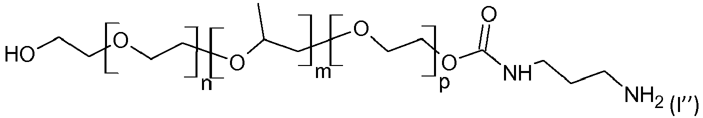 or of formula (II') 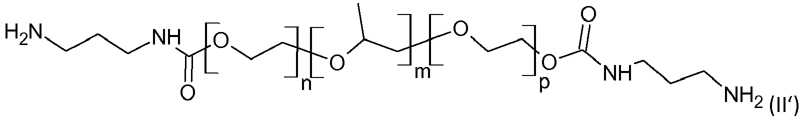 wherein in formulae (I') (I") and (II')
n is independently an integer from 130-150, preferably 139-145, more preferably 141;
m is independently an integer from 40 to 50, preferably 43-46 more preferably 44;
p is independently an integer from 130-150, preferably 139-145, more preferably 141; an NH₂ group is protonated and n, m and p are the average mole numbers;
or a mixture of compounds of formulae (I') (I") and (II').

6. The composition according to claims 1-5 wherein the composition has a pH ranging from 7 to 7.6, preferably from 7.1 to 7.4.

7. The composition according to claims 1-6 which is thermo-gelatable.

8. The composition according to claims 1-7 which becomes a gel at a temperature ranging from 28-37 °C, preferably ranging from 35-36°C.

9. The composition according to claims 1-8 wherein components 1) and 2) are in the ionic forms thereof partially or totally.

10. The composition as defined in any of claims 1-9, wherein the ophthalmic surgery is a vitrectomy.

## Patentansprüche

1. Gelierbare Sol-Zusammensetzung zur Verwendung in der Augenchirurgie, wobei die Zusammensetzung umfasst:
1) Polysaccharid mit einer deprotonierten Säureeinheit, wobei das Polysaccharid vorzugsweise Hyaluronsäure (HA) oder ein Derivat davon, ausgewählt aus der Gruppe bestehend aus Estern, Carbamaten, Amiden und mit 1-Ethyl-3-(3-dimethylaminopropyl)carbodiimid-hydrochlorid (EDC) funktionalisierter Hyaluronsäure, ist;
2) Mono- und/oder bi-terminiertes Aminotriblockcopolymer, ausgewählt aus mono-Amino-terminiertem Poly(ethylenglycol)-block-poly(propylenglycol)-*block*-poly(ethylenglycol), bi-Amino-terminiertem Poly(ethylenglycol)-*block*-poly(propylenglycol)-*block*-poly(ethylenglycol) oder Mischungen davon, wobei eine Aminogruppe des Triblockcoplymers protoniert ist; und
3) Wasser oder physiologische Kochsalzlösung oder ein wässriges Puffersystem.

2. Zusammensetzung gemäß Anspruch 1, wobei die Bestandteile 1) und 2) zusammen 18 bis 30 Gew.-%, vorzugsweise 18 bis 25 Gew.-%, stärker bevorzugt 20 Gew.-%, der Zusammensetzung ausmachen.

3. Zusammensetzung gemäß einem der vorangehenden Ansprüche, wobei die Bestandteile 2) und 1) in einem Gewichtsverhältnis im Bereich von 80:20 bis 97:3, vorzugsweise 93:7 bis 96:4, stärker bevorzugt 94:6 bis 95:5, noch stärker bevorzugt 94,7:5,3, vorliegen.

4. Zusammensetzung gemäß einem der vorangehenden Ansprüche, wobei der Bestandteil 2) die Formel hat: 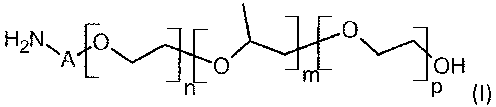 wobei
A aus -CO-C₂-C₆-Alkylen oder -CO-C₂-C₆-Alkylen-NH-C₂-C₆-Alkylen ausgewählt ist, wobei C₂-C₆-Alkylen vorzugsweise Propylen oder Ethylen ist und wobei in A die -CO-Gruppe mit dem Polymerteil der Formel (I) verbunden ist;
n unabhängig eine ganze Zahl von 130-150, vorzugsweise 139-145, stärker bevorzugt 141, ist;
m unabhängig eine ganze Zahl von 40 bis 50, vorzugsweise 43-46, stärker bevorzugt 44, ist;
p unabhängig eine ganze Zahl von 130-150, vorzugsweise 139-145, stärker bevorzugt 141, ist;
die NH₂-Gruppe protoniert ist und n, m und p die durchschnittlichen Molzahlen sind;
oder die Formel (I*) hat: 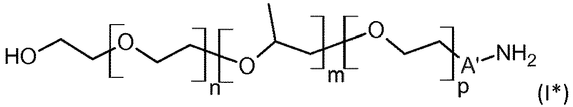 wobei
A' unabhängig aus -O-CO-C₂-C₆-Alkylen oder -O-CO-NH-C₂-C₆-Alkylen ausgewählt ist, wobei C₂-C₆-Alkylen vorzugsweise Propylen oder Ethylen ist, wobei in A' die -O-CO-Gruppe mit dem Polymerteil der Formel (I*) verbunden ist;
n unabhängig eine ganze Zahl von 130-150, vorzugsweise 139-145, stärker bevorzugt 141, ist;
m unabhängig eine ganze Zahl von 40 bis 50, vorzugsweise 43-46, stärker bevorzugt 44, ist;
p unabhängig eine ganze Zahl von 130-150, vorzugsweise 139-145, stärker bevorzugt 141, ist;
die NH₂-Gruppe protoniert ist und n, m und p die durchschnittlichen Molzahlen sind;
oder die Formel (II) hat: 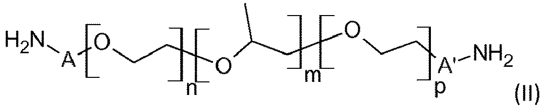 wobei
A aus -CO-C₂-C₆-Alkylen oder -CO-NH-C₂-C₆-Alkylen ausgewählt ist, wobei C₂-C₆-Alkylen vorzugsweise Propylen oder Ethylen ist, wobei in A die -CO-Gruppe mit dem Polymerteil der Formel (II) verbunden ist;
A' aus -O-CO-C₂-C₆-Alkylen oder -O-CO-NH-C₂-C₆-Alkylen ausgewählt ist, wobei C₂-C₆-Alkylen vorzugsweise Propylen oder Ethylen ist, wobei in A' die -O-CO-Gruppe mit dem Polymerteil der Formel (II) verbunden ist;
n unabhängig eine ganze Zahl von 130-150, vorzugsweise 139-145, stärker bevorzugt 141, ist;
m unabhängig eine ganze Zahl von 40 bis 50, vorzugsweise 43-46, stärker bevorzugt 44, ist;
p unabhängig eine ganze Zahl von 130-150, vorzugsweise 139-145, stärker bevorzugt 141, ist;
die NH₂-Gruppe protoniert ist und n, m und p die durchschnittlichen Molzahlen sind;
oder eine Mischung der Verbindungen der Formeln (I), (I*) und (II) ist.

5. Zusammensetzung gemäß einem der vorangehenden Ansprüche, wobei der Bestandteil 2) die Formel (I') hat: 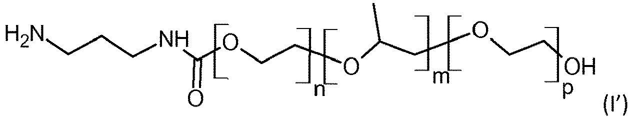 oder die Formel (I") hat: 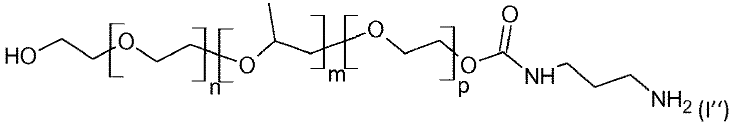 oder die Formel (II') hat: 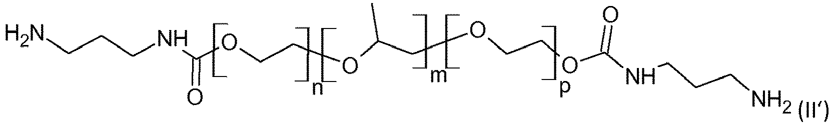 wobei in den Formeln (I'), (I") und (II')
n unabhängig eine ganze Zahl von 130-150, vorzugsweise 139-145, stärker bevorzugt 141, ist;
m unabhängig eine ganze Zahl von 40 bis 50, vorzugsweise 43-46, stärker bevorzugt 44, ist;
p unabhängig eine ganze Zahl von 130-150, vorzugsweise 139-145, stärker bevorzugt 141, ist;
die NH₂-Gruppe protoniert ist und n, m und p die durchschnittlichen Molzahlen sind;
oder eine Mischung der Verbindungen der Formeln (I'), (I") und (II') ist.

6. Zusammensetzung gemäß der Ansprüche 1-5, wobei die Zusammensetzung einen pH-Wert im Bereich von 7 bis 7,6, vorzugsweise von 7,1 bis 7,4, hat.

7. Zusammensetzung gemäß der Ansprüche 1-6, die thermisch gelierbar ist.

8. Zusammensetzung gemäß der Ansprüche 1-7, die zu einem Gel bei einer Temperatur im Bereich von 28-37 °C, vorzugsweise im Bereich von 35-36°C, wird.

9. Zusammensetzung gemäß der Ansprüche 1-8, wobei die Bestandteile 1) und 2) teilweise oder vollständig in deren ionischen Formen vorliegen.

10. Zusammensetzung, wie in einem der Ansprüche 1-9 definiert, wobei die Augenoperation eine Vitrektomie ist.

## Revendications

1. Composition de sol gélifiable pour utilisation en chirurgie ophtalmique, dans laquelle la composition comprend:
1) un polysaccharide ayant une fraction acide déprotonée, dans laquelle de préférence le polysaccharide est l'acide hyaluronique (HA) ou un dérivé de celui-ci choisi dans le groupe composé d'esters, de carbamates, d'amides et d'acide hyaluronique fonctionnalisés avec du chlorhydrate de 1-éthyle-3-(3-diméthylaminopropyle) carbodiimide (EDC);
2) un copolymère triséquencé amino à terminaison mono et/ou bis choisi parmi le poly (éthylène glycol)-*bloc*-poly(propylène glycol) -*bloc*-poly(éthylène glycol) à terminaison mono-amino, un poly(éthylène glycole)-*bloc*-poly(propylène glycol)-*bloc*-poly(éthylène glycol) à terminaison bis-amino ou un mélange de ceux-ci, dans lequel un groupe amino du copolymère triséquencé est protoné; et
3) de l'eau ou une solution saline physiologique ou un système tampon aqueux.

2. Composition selon la revendication 1, dans laquelle les composants 1) et 2) ensemble représentent de 18% à 30% en poids, de préférence de 18% à 25% en poids, plus préférablement de 20% en poids de la composition.

3. Composition selon l'une quelconque des revendications précédentes, dans laquelle les composants 2) et 1) sont dans un rapport pondéral allant de 80:20 à 97:3, de préférence de 93:7 à 96:4, plus préférablement de 94:6 à 95:5, encore plus préférablement 94,7: 5,3.

4. Composition selon l'une quelconque des revendications précédentes, dans laquelle le composant 2) est de formule 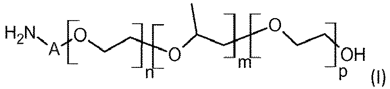 dans laquelle
A est choisi parmi l'alkylène en -CO-C₂-C₆ ou l'alkylène en -CO-C₂-C₆, l'alkylène en -NH-C₂-C₆, de préférence l'alkylène en C₂-C₆ est le propylène ou l'éthylène et dans laquelle A dans le groupe -CO- est lié à la partie polymère de formule (I);
n est indépendamment un nombre entier de 130-150, de préférence de 139-145, plus préférablement 141;
m est indépendamment un nombre entier de 40 à 50, de préférence de 43 à 46, plus préférablement 44;
p est indépendamment un nombre entier de 130-150, de préférence de 139-145, plus préférablement 141; le groupe NH₂ est protoné et n, m et p sont les nombres molaires moyens;
ou de formule (I*) 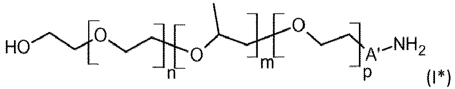 dans laquelle
A' est choisi parmi l'alkylène en -O-CO-C₂-C₆ ou l'alkylène en -O-CO-NH-C₂-C₆, de préférence l'alkylène en C₂-C₆ est le propylène ou l'éthylène, dans laquelle dans A' le groupe -O-CO- est lié à la partie polymère de formule (I*);
n est indépendamment un nombre entier de 130-150, de préférence de 139-145, plus préférablement 141;
m est indépendamment un nombre entier de 40 à 50, de préférence de 43 à 46, plus préférablement 44;
p est indépendamment un nombre entier de 130-150, de préférence de 139-145, plus préférablement 141; le groupe NH₂ est protoné et n, m et p sont les nombres molaires moyens;
ou de formule (II) 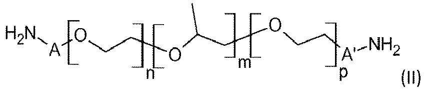 dans laquelle
A est choisi parmi l'alkylène en -CO-C₂-C₆ ou l'alkylène en -CO-NH-C₂-C₆, de préférence l'alkylène en C₂-C₆ est le propylène ou l'éthylène, dans laquelle A dans le groupe -CO- est lié à la partie polymère de formule (II);
A' est choisi parmi l'alkylène en -O-CO-C₂-C₆ ou l'alkylène en -O-CO-NH-C₂-C₆, de préférence l'alkylène en C₂-C₆ est le propylène ou l'éthylène, dans laquelle dans A' le groupe -O-CO- est lié à la partie polymère de formule (II);
n est indépendamment un nombre entier de 130-150, de préférence de 139-145, plus préférablement 141;
m est indépendamment un nombre entier de 40 à 50, de préférence de 43 à 46, plus préférablement 44;
p est indépendamment un nombre entier de 130-150, de préférence de 139-145, plus préférablement 141; le groupe NH₂ est protoné et n, m et p sont les nombres molaires moyens;
ou un mélange de composés de formules (I), (I*) et (II).

5. Composition selon l'une quelconque des revendications précédentes, dans laquelle le composant 2)
est de formule (I') 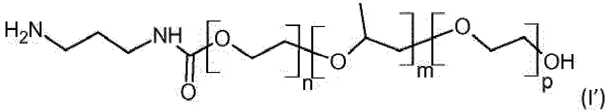 ou de formule (I") 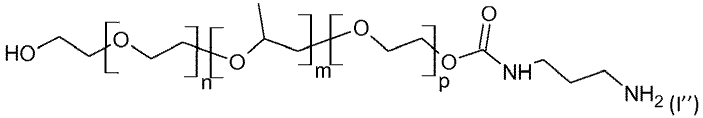 ou de formule (II') 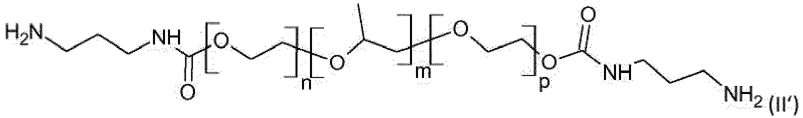 où dans les formules (I'), (I") et (II')
n est indépendamment un nombre entier de 130-150, de préférence de 139-145, plus préférablement 141;
m est indépendamment un nombre entier de 40 à 50, de préférence de 43 à 46, plus préférablement 44;
p est indépendamment un nombre entier de 130-150, de préférence de 139-145, plus préférablement 141; le groupe NH₂ est protoné et n, m et p sont les nombres molaires moyens;
ou un mélange de composés de formules (I), (I*) et (II').

6. Composition selon les revendications 1 à 5, dans laquelle la composition présent un pH allant de 7 à 7,6, de préférence de 7,1 à 7,4.

7. Composition selon les revendications 1 à 6, qui est thermo-gélifiable.

8. Composition selon les revendications 1 à 7, qui se transforme en gel à une température allant de 28-37°C, de préférence allant de 35 à 36°C.

9. Composition selon les revendications 1 à 8, dans laquelle les composants 1) et 2) se présentent sous des formes ioniques de ceux-ci partiellement ou totalement.

10. Composition selon l'une quelconque des revendications 1 à 9, dans laquelle la chirurgie ophtalmique est une vitrectomie.
